# EUROPEAN PATENT SPECIFICATION

(11) **EP 3 996 801 B1**
(45) Date of publication and mention of the grant of the patent: **24.01.2024**
(21) Application number: 20742411.0
(22) Date of filing: 13.07.2020
(51) Int. Cl.: A61N 1/05, A61N 1/36, A61N 1/04

(54) **MEDICAL DEVICE**
MEDIZINISCHE VORRICHTUNG
DISPOSITIF MÉDICAL

(30) Priority: 11.07.2019 GB 201909984
(43) Date of publication of application: 18.05.2022
(73) Proprietor: Cambridge Enterprise Limited, Cambridge, Cambridgeshire CB2 1TN (GB)
(72) Inventor: PROCTOR, Christopher, Cambridge Cambridgeshire CB3 0GT (GB); BARONE, Damiano Giuseppe, Cambridge Cambridgeshire CB3 0GT (GB); CURTO, Vincenzo, Cambridge Cambridgeshire CB3 0GT (GB); WOODINGTON, Ben, Cambridge Cambridgeshire CB3 0GT (GB); MALLIARAS, George, Cambridge Cambridgeshire CB3 0GT (GB)
(74) Representative: J A Kemp LLP
(86) International application number: PCT/GB2020/051684
(87) International publication number: WO 2021/005382

(56) References cited:
- US-A1- 2007 027 514
- US-B1- 6 266 568
- US-B1- 6 368 349

## Description

The present invention relates to a medical device having an electrode array. The invention is of particular relevance to implantable devices, for example those interfacing with biological tissue such as the nervous system for purposes such as recording cellular activity for scientific or diagnostic purposes, electrical stimulation, pain management, rehabilitation, and brain-machine interfaces

Various medical devices can incorporate electrode arrays, either for actively stimulating tissue or for passively sensing (or a combination of the two). In recent years, implantable bioelectronics devices for treating and diagnosing disease have emerged as a prominent component of modern healthcare. When used for the treatment of chronic disorders, implantable bioelectronics devices make use of electric pulses to, for example, restore the physiological function of organs (as in heart pacemakers and cochlear implants) or alleviate chronic side effect of neurodegenerative syndromes (as in deep brain stimulators (DBS) to stop tremor in Parkinson's disease). In addition to this, implantable bioelectronic devices are used clinically for acute (up to three weeks) recording/mapping of neural activity in patients undergoing surgical brain resection of epileptogenic tissue.

However, the risk and cost of the surgery to implant devices remains a limiting factor.

By way of a particular example, clinically available spinal cord stimulators (SCSs) are used for pain management. To date, SCS devices have been primarily used for chronic pain management caused by failed back surgery syndrome and angina, among other disorders. Such devices are implanted in the extradural space between the spinal cord and the spine. They work by creating local electric fields that interfere with the transmission of nerve signals from their source to where they are registered in the brain.

There are two types of commercially available stimulators: the linear and the paddle designs. The linear array of electrodes (e.g. electrodes arranged sequentially on a single wire) can be implanted percutaneously, through a needle, in a simple and cost-effective procedure. Unfortunately, the benefit of easy implantation for this type of device is negated by both a very limited spatial resolution and poor anatomical targeting capability these slender wire-like devices can provide. In contrast, the paddle are millimetres thick, presenting electrodes e.g. in columns over a broader 'paddle-shaped' area than a single wire device, and thus cover a larger surface area of the spinal cord and provide a more specific and effective area for the spinal electric stimulation. However, implantation of the bulkier paddle designs cannot be done so simply, and so requires a risky and expensive surgical procedure under general anaesthesia.

Inflatable devices are known but often suffer from a number of disadvantages. In particular they may require significant space to deploy from an uninflated state to an inflated state, and/or may have undesirable side-effects due to the expansion of the device caused by the inflation process. Packaging of inflatable devices has also been a challenge as it requires the implant to be sufficiently flexible to be rolled or folded into a compressed state that is small enough for percutaneous insertion. In contrast, clinically available devices such as spinal cord stimulators and electrocorticography arrays as well as other proposed inflatable devices have components such as thick metal electrodes or silicon chips that are too stiff to elastically bend at a sufficiently small radius.

US 6,368,349B discloses a neural prosthesis for implantation within an eye. The prosthesis includes a foldable substrate and at least one electronic component supported by the substrate. At least one microchannel is disposed within the substrate. Upon inflation, the foldable substrate will unfold to provide for close contact of the electronic component with neural tissue, thus facilitating surgical implantation through a narrow incision, yet allowing the unfolded device to cover a sufficiently large portion of the patient's retina to provide useful vision.

As such, the existing options for such medical devices are not satisfactory. The present invention aims to at least partially address this problem.

A first aspect of the present invention provides a medical device, comprising: a flexible electrode array having a bend radius of no more than about 2mm; and a fluidic component, wherein the fluidic component is fluidically actuatable to cause the fluidic component to change configuration; wherein the fluidic component and the flexible electrode array are configured such that a change in configuration of the fluidic component causes a change in configuration of the flexible electrode array.

The electrode array of the above aspect is extremely flexible, having a bend radius of no more than 2mm, preferably no more than 1.5mm and more preferably no more than 1mm. Bend radius, which is measured to the inside curvature, is the minimum radius that a component (in this case the electrode array) can be bent in at least one direction without damaging it. The bend radius as defined here refers to elastic deformation as opposed to plastic deformation such that an electrode array bent under an applied force to a radius greater than the minimum bend radius would return at least part way to its original shape with the removal of the applied force. In other words, the electrode array in the device of this aspect can be bent to an inside curvature of 2mm, for example by rolling when the device is being arranged for insertion into a patient, and subsequently deployed (e.g. unrolled) to an expanded, less bent configuration (e.g. a substantially planar configuration) and still function exactly as it did prior to bending.

Preferably the flexible electrode array has a bend radius of no more than about 1.5mm, more preferably no more than about 1mm, more preferably no more than about 0.5mm. Lower bend radii for the flexible electrode can allow the electrode array to be rolled into tighter (and thus thinner) cylindrical structures for deployment, whilst still retaining the functionality of the electrode array when the device is deployed by a change in configuration of the fluidic component.

The device may have a proximal section and a distal section, the flexible electrode array and the fluidic component being arranged in the distal section. The distal section may have a bend radius of no more than about 2mm in a first direction (and preferably smaller, for example 1.5mm, 1mm or 0.5mm or less). It is generally the distal section of the device which needs to deploy in order for the electrode array to be arranged to perform its function, for example when implanted in a patient. Thus it may be the distal section which changes configuration on actuation of the fluidic component. Other parts of the device, such as connectors to external components such as tubes and wires which connect the device to further apparatus such as an implanted pulse generator and may be rigid (and may be required to be rigid) can be arranged in the proximal section and thus not affect the ability of the distal section to change configuration on fluidic actuation.

In certain arrangements, the medical device, and in particular the distal section of the device, may have different properties in different directions. For example, the distal section may have a bend radius in a second direction which is orthogonal to said first direction which is more than the bend radius in the first direction. This may apply to the whole of the distal section or to particular parts of the distal section (such as the flexible electrode array). Such variations in properties could for instance take the form of a device that is relatively stiff or inelastic along the axis of insertion to aid in positioning of the implant while still being sufficiently flexible in the orthogonal directions such that the device can be rolled or otherwise compressed to allow for implantation through a small incision.

In certain embodiments the medical device is elongate and the first direction is substantially perpendicular to the longitudinal axis of the device. This can allow the flexible electrode array and/or distal section to be packaged in a manner which reduces the thickness of the device (for example in order to pass through a small incision, aperture, lumen or catheter during deployment of the device) and then deployed by fluid actuation into a larger configuration when in the desired position. Often it is desirable to reduce the thickness dimensions of the device for deployment through as small a gap as possible, whilst it is less important to change or reduce the length dimension of the device as this does not affect the size of incision, aperture, lumen or catheter needed.

In certain embodiments the device has a removable support element. The removable support element may provide rigidity to the device in one or more directions in order to assist with deployment of the device. For example the removable support element may be a stiff element which extends along some or all of the longitudinal extent of the device in order to maintain rigidity of the device during deployment (e.g. by preventing "crumpling" of the device as it is urged into a patient).

In certain embodiments the device is configured such that, when the removable support element is removed, the distal section has a bend radius of no more than about 2mm (and preferably less, for example, 1.5mm, 1mm, 0.5mm or less) in each of the first and second directions. Thus the removable support element may provide temporary or removable support or rigidity to the device and can then be removed once that support is no longer needed.

A further aspect of the present invention provides a medical device comprising: a flexible electrode array; and a fluidic component, wherein the fluidic component is fluidically actuatable to cause the fluidic component to change configuration; wherein the fluidic component and the flexible electrode array are configured such that a change in configuration of the fluidic component causes a change in configuration of the flexible electrode array, further wherein the flexible electrode array and the fluidic component are arranged such that a change in configuration of the fluidic component causes the flexible electrode array to transition between a compressed configuration and an expanded configuration having a greater projected surface area than the compressed configuration.

In certain embodiments, in the compressed configuration the flexible electrode array and, optionally, the fluidic component, is rolled. Rolling the flexible electrode array makes good use of the available cross-section in a limited diameter incision, aperture, lumen or catheter.

Rolling is also facilitated by a device having a small bend radius in the portions which change configuration.

In certain embodiments the transition between the compressed configuration and the expanded configuration includes unrolling of the flexible electrode array. This unrolling may be about an axis parallel to the longitudinal extent of the device and/or about an axis perpendicular to the direction in which the electrode array and/or the fluidic component has a small bend radius (e.g. the first direction in the above aspect).

In the compressed configuration, the flexible electrode array and/or fluidic component may be substantially cylindrical and/or have a circular cross-section. Compressing the electrode array and/or fluidic component into a cylindrical form or such that it has a circular cross-section optimises the packing of the device into the available diameter for insertion into a patient.

In the expanded configuration, the flexible electrode array may be substantially planar. Preferably, in the expanded configuration, the electrode array conforms to organ or tissue that it is intended to interact with, either in an active or passive manner. Such conformation may have a degree of curvature, but the overall configuration of the device may still be substantially planar compared, for example, to the compressed configuration.

Preferably, in the expanded configuration, the medical device has a thickness of no more than 5mm, more preferably no more than 3mm, more preferably no more than 2mm, and in some embodiments may be 1mm or less. The thickness of the device can be important to ensure reduced or minimal interaction with the surrounding tissue. Whilst expansion of the electrode array in the deployed configuration such that it has a greater projected area than in the compressed configuration is desirable for the electrode array to deploy across a treatment or detection area that is larger than that in which it is inserted into the patient, expansion in the thickness direction is generally less desirable and should be reduced and avoided if possible.

In certain embodiments, the electrode array and/or fluidic component are arranged such that the electrode array can retain its deployed shape even if the fluidic component is subsequently partly or wholly deflated. This can assist in reducing the thickness of the device in its deployed configuration. In such embodiments, the thickness of the medical device in the expanded or deployed configuration may be no more than 0.5mm, preferably no more than 0.2mm and more preferably no more than 0.1mm.

Preferably the medical device is arranged to limit expansion in the thickness of the device during changes in configuration.

In certain embodiments the medical device further comprises a constraining layer which is arranged substantially parallel to the fluidic component and includes one or more portions of stiff or inelastic or low elasticity material which are arranged to prevent or limit expansion of the fluidic component in the thickness direction of the device during changes in configuration. Reference to "inelastic" in the following description will be understood to include materials with low levels of elasticity. Lower levels of elasticity are preferred for the function of limiting expansion, but some degree of elasticity may be desirable for other purposes.

The portions of stiff material may include a plurality of strips which are arranged substantially parallel to each other and wherein the parts of the constraining layer between said strips are more flexible.

Alternatively or additionally, the portions of stiff material may be arranged so as not to impede the change of configuration in directions other than the thickness direction.

In certain embodiments, the limitation on vertical expansion is achieved by incorporating an inelastic material into one or more layers above and/or below the fluidic component. This relatively inelastic material may resist deformation and therefore restrict expansion in the vertical direction. Likewise, a flexible but inelastic material above and/or below the fluidic component would prevent the fluidic chamber from stretching or ballooning to a larger volume. Such a material system could for instance take the form of thin layers of parylene-C or polyimide with or without layers of silicone.

Any such inelastic material can also be specifically configured to take account of the requirements for the overall flexibility of the device for the deployment process. This could, for example, be achieved by providing strips of stiff material with regions of flexible material between them, the strips being oriented perpendicular to the direction of unrolling or unfurling of the device during deployment, such that the flexible material ensures that the device as a whole is still sufficiently flexible to deploy, while the stiff strips prevent or reduce the vertical expansion by increasing the force needed to cause such expansion.

Alternatively or additionally, a material could be used to form a layer in the device above and/or below the fluidic component which has anisotropic properties, such that it is flexible in the direction of rolling/unrolling, but stiff in the perpendicular (e.g. longitudinal) direction.

In certain embodiments the fluidic component comprises a fluidic channel extending within the fluidic component and the fluidic component further comprises at least one tie which joins opposing sides of the fluidic channel so as to prevent or limit expansion of the fluidic channel in the thickness direction of the device during changes in configuration.

The tie(s) can be manufactured as part of the channel itself, or may be formed by bonds or welds between top and bottom layers of the fluidic channel. The ties may be spot joins, with a plurality of such joins distributed along the channel, or may be contiguous along all or part of the channel.

Alternatively or additionally the fluidic component may include a plurality of independently inflatable chambers wherein the chambers are sized so as to prevent or limit expansion of the fluidic channel in the thickness direction of the device during changes in configuration. If the individual chambers or sections of the fluidic component are sufficiently small in cross section, then vertical expansion may be prevented or restricted. Thus an overall design of the fluidic component in which a fluidic channel which is small in cross-section may be provided. A plurality of such channels may be arranged in parallel to each other and be joined at either end.

Alternatively or additionally the fluidic component further includes a pressure valve arranged fluidically between a first of said independently inflatable chambers and a second of said independently inflatable chambers, said pressure such that fluid will not pass from the first chamber to the second chamber until a predetermined fluid pressure is reached in the first chamber. The vertical expansion of the device can then be limited by the design of the geometry of the chambers and the pressure limits set by the valves.

A further aspect of the present invention provides a medical device comprising: a flexible electrode array; and a fluidic component, wherein the fluidic component is fluidically actuatable to cause the fluidic component to change configuration; wherein the fluidic component and the flexible electrode array are configured such that a change in configuration of the fluidic component causes a change in configuration of the flexible electrode array, wherein the device has a proximal section and a distal section, the flexible electrode array and the fluidic component being arranged in the distal section, the device further comprising: a fluidic connector in fluid communication with the fluidic component and an electrical connector in electrical contact with the electrode array, said connectors being provided in the proximal section of the device for connection of the fluidic component and the electrode array to external devices.

The distal section of the device may be more flexible in at least one direction than the proximal section.

Thus the distal section of the device may contain the flexible and re-configurable components such as the fluidic component and the electrode array, whilst less flexible (or inflexible) components such as connectors can be located in the proximal end which, preferably, does not change configuration during deployment of the device.

The terms distal section and proximal section are intended to refer to the relative arrangement of the components described in this aspect. In particular, in certain embodiments, it is not envisaged that the device itself includes any wires or other connectors (e.g. tubes) which serve to connect the device to further apparatus or devices (such as controllers and/or fluid and/or power sources) external to, or at the skin level of the patient after insertion of the device. Thus the proximal section of the device may solely contain the components necessary to make connections to such items.

In such arrangements, the proximal section of the device may form a relatively small proportion of the device as a whole, for example no more than 20%, preferably no more than 15%, more preferably no more than 10%, more preferably no more than 5% of the total volume of the device in the deployed state (such that the distal section having the active components comprises 80%, 85%, 90% or 95% of the volume of the device respectively).

The device may further comprise a conductive connector connecting the electrode array to the electrical connector and a first sheath which surrounds the conductive connector. The first sheath may be electrically insulating.

In certain embodiments, the device may further comprise a fluid channel connecting the fluidic component to the fluidic connector, wherein the first sheath also surrounds the fluid channel.

The device may further comprise a second, removable sheath surrounding the flexible electrode array, the fluidic component, and the first sheath. The second sheath may serve to protect the fluidic component, electrode array and the connector(s) during insertion of the device into a patient and/or to prevent deformation of the device during insertion.

In particular, the flexible electrode array and the fluidic component may arranged in a compressed configuration within the second sheath, and the device is arranged such that actuation of the fluidic component after removal of the sheath causes the fluidic component and the flexible electrode array to change to an expanded configuration having a greater projected surface area than the compressed configuration.

The internal diameter of the second sheath is preferably 1 cm or less, optionally 5 mm or less, further optionally 2 mm or less.

According to another aspect of the invention, there is provided a medical device, comprising one or more of: a flexible electrode array; and a fluidic component, wherein the fluidic component is fluidically actuatable to cause the fluidic component to change configuration; wherein the fluidic component and the flexible electrode array are configured such that a change in configuration of the fluidic component causes a change in configuration of the flexible electrode array.

Optionally, the medical device is a bioelectric implant. The bioelectric implant may be an active implant, such as a spinal cord stimulator. The bioelectric implant is a passive implant, such as an electrocorticography sensor.

Optionally, the flexible electrode array comprises electrodes provided on a flexible substrate. The flexible substrate may be 500 µm thick or less, optionally 200 µm thick or less, further optionally 100 µm thick or less, further optionally 50 µm thick or less, further optionally 25 µm thick or less, further optionally 10 µm thick or less and still further optionally 5 µm thick or less. The flexible substrate may be made of a polymeric material, optionally a thermoplastic, and optionally comprising one or more of a poly-urethane, a silicone, a parylene, a polyimide, a polyamide, a cyclic olefin polymer, a cyclic olefin copolymer, a polyacrylate, polyethylene terephthalate and/or an epoxy.

Optionally, the flexible substrate comprises the fluidic component.

Optionally, the fluidic component comprises a fluidic inlet for supplying fluid into the fluidic component.

Optionally, the fluidic component comprises a fluidic channel connected to the fluidic inlet, the channel extending within the fluidic component.

Optionally, the fluidic channel is not rigid.

Optionally, the fluidic channel has: a maximum uninflated width dimension of 5 mm or less, optionally 3 mm or less, further optionally 1 mm or less, further optionally 500µm or less, further optionally 100µm or less, further optionally 50µm or less, and still further optionally 5µm or less; and/or a maximum inflated thickness of no more than 5 mm, optionally no more than 2 mm, further optionally no more than 1 mm, and still further optionally no more than 500µm.

Optionally, the fluidic component is actuated by supplying fluid to the fluidic channel.

Optionally, the fluidic channel has a branching and/or symmetrical structure within the fluidic component.

Optionally, the medical device can be configured to a first configuration have diameter of 1 cm or less, optionally 5 mm or less, further optionally 2 mm or less, and still further optionally 1 mm or less.

Optionally, the medical device can be actuated from said first configuration to an expanded configuration having a greater projected surface area than the first configuration by the fluidic actuation.

Optionally, the device is configured such that the fluidic actuation causes the fluidic component to unfurl or unfold, thereby unfurling or unfolding the flexible electrode array.

Optionally, the fluidic component is separate or separable from the flexible electrode array.

The fluidic component and the flexible electrode array in any of the above devices may be separate or separable. This can allow the fluidic component to be used in the delivery and deployment of the electrode array, but then be withdrawn leaving only the array in situ in the patient. This can significantly reduce the size of the device retained within the patient, which may provide for lower levels of disruption to surrounding tissue and organs (and thus potentially fewer side-effects from the implantation of the device).

The medical device of any of the above aspects may include one or more components which are imageable by X-ray such as a strip of a polymer material infused with BaSO₄. This allows the position of the device to be checked and/or monitored during and/or after the device has been deployed in a patient.

Unless indicated otherwise, any of the features (including the optional or preferred features) described in relation to one of the above aspects are equally applicable in combination with the medical devices according to any of the other above-described aspects.

According to a further aspect, not part of the claimed invention, there is provided a method of using a medical device according to any of the previously described aspects (including some, all or none of the optional and preferred features of those aspects), the method comprising at least one of: supplying fluid to the fluidic component, so as to cause a change in configuration of the fluidic component; wherein the fluidic component, as it is changing configuration, causes a change in configuration of the flexible electrode array.

Optionally, the method further comprises removing the fluidic component from the flexible electrode array.

Optionally, the method further comprises: configuring the bioelectric implant in a first configuration, suitable for deployment; deploying the bioelectric implant; fluidically actuating the bioelectric implant so as to change the bioelectric implant from a first configuration into a second configuration.

The bioelectric implant can be deployed percutaneously, or through a burr hole, the burr hole optionally being 20 mm or less in diameter, further optionally 10 mm or less, further optionally 5 mm or less, and still further optionally 2 mm or less.

Optionally, the step of actuating further comprises bringing the electrodes of the bioelectric implant into contact or proximity with a target tissue.

The invention is described below, by way of example, with reference to the accompanying figures in which:
Fig. 1 is drawing of a medical device comprising a flexible electrode array and a fluidics component;
Fig. 2 shows examples of (A) longitudinal and (B) lateral unfolding/unfurling of a medical device such as presented in Fig. 1;
Fig 3 illustrates various patterns that may be used for the fluidics component of the medical device;
Fig. 4 illustrates a medical device according to an embodiment of the present invention;
Fig. 5 illustrates a medical device according to an embodiment of the present invention and the sheathing of certain components of the device;
Fig. 6 illustrates the deployment of the device to a spinal cord location;
Fig. 7 shows, schematically, the cross-sectional configuration of a fluid channel within a medical device;
Fig. 8 illustrates the steps of a protocol for creating a flexible electrode array;
Fig. 9 illustrates the steps of an alternative protocol for creating a flexible electrode array;
Fig. 10 illustrates the steps of a protocol for combining a fluidics component with a flexible electrode array;
Fig. 11 illustrates the steps of an alternative protocol for combining a fluidics component with a flexible electrode array; and
Fig. 12 illustrates the steps of a further alternative protocol for combining a fluidics component with a flexible electrode array.

The present disclosure relates to medical devices, particularly, implantable bioelectronic devices, which incorporate a fluidic component (it being understood that: a 'fluid' can be any of a liquid, gas, gel or foam or combinations thereof; a `fluidic component' covers both pneumatic and hydraulic components, as well as those actuated by gels or foams, or combinations thereof; and `fluidically actuatable' means that the component may be actuated by any of a liquid, gas, gel or foam or combinations thereof) that can be used to actuate the unfolding/unrolling of said device post implantation. By providing a flexible device that can be rolled up prior to implantation, the device may be deployed relatively simply, e.g. percutaneously. Once deployed, by being able to control the unfolding, the device can be positioned as needed and have a relatively large active surface area compared to the size of the device in the rolled configuration.

Such devices address the critical shortcomings of other implant technologies, such as those used in spinal cord stimulation (SCS) discussed above, in terms of reducing surgical invasiveness of implantation allowing for percutaneous implantation of large implants.

In the discussion below, for ease of reference, the term "gathered" or "compressed" configuration is used to contrast with "expanded" configuration. The skilled person will understand that the gathered configuration can encompass any form or combination of folding, rolling, pleating etc.

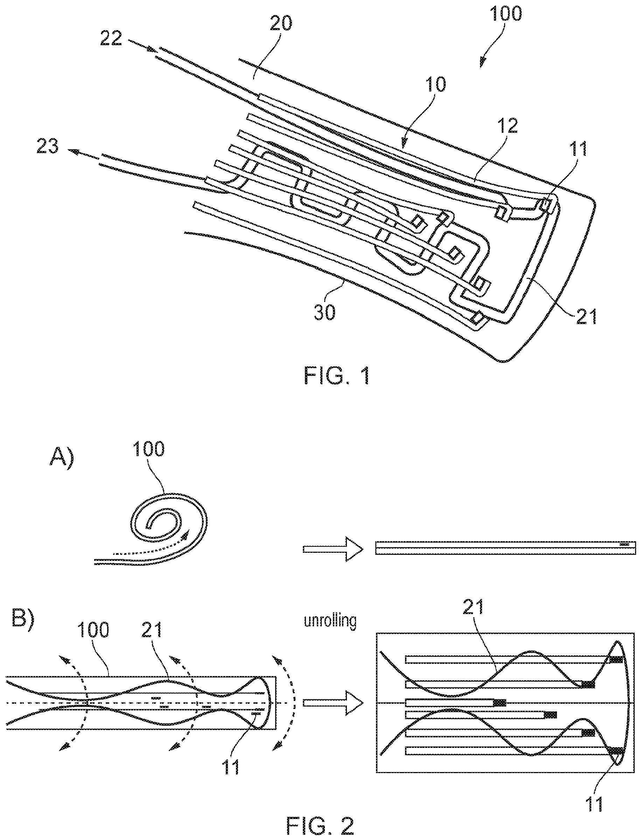

Figure 1 illustrates a medical device 100. The medical 100 may be a bioelectric implant, for example. The bioelectric implant 100 may be an active implant, such as a spinal cord stimulation (SCS) device. Alternatively, the bioelectric implant may be a passive implant, such as an electrocorticography sensor. In other applications, device 100 may have both active and passive functions. Other applications for such devices 100 include for use in peripheral nerve implants or recording/stimulating muscle activity.

The medical device 100 comprises a flexible electrode array 10. The flexible electrode array 10 comprises electrodes 11 connected to conductive lines 12, provided on a flexible substrate 30. By way of non-limiting example, the flexible electrode array may be around 5 µm thick. The electrode array 10 is flexible so that it can change in configuration in response, actuated by the he fluidic component 20, as explained below. As such, herein, the phrase "flexible electrode array" is used to mean an array that can undergo such changes in configuration. That includes arrays which are entirely flexible, or semi-flexible (e.g. including some parts or features which are rigid or more rigid than other more flexible parts, provided they can still undergo the change in configuration actuated by the fluidic component).

The medical device 100 also includes a fluidic component 20. The fluidic component 20 is fluidically actuatable to cause the fluidic component 20 to change configuration, as discussed below. The fluidic component 20 can be a microfluidic component. In other arrangements, there may be one or more fluidic components, but a single fluidic component 20 is illustrated for ease of understanding.

The fluidic component 20 and the flexible electrode array 10 are configured such that a change in configuration of the fluidic component 20 causes a change in configuration of the flexible electrode array 10.

In the illustrated embodiment, the change in configuration of the fluidic component 20 causes a change in configuration of the electrode array 10 because the substrate 30 of the electrode array 10 comprises the fluidic component 20. As such, the fluidic component 20 and the electrode array 10 are integrally connected.

However, in other configurations, the fluidic component 20 may be separate, or separable from, the electrode array 10. Indeed, as will be understood from the following description, the fluidic component 20 and the electrode array 10 may not be connected by any other means than the gathering of the components together before implantation. The benefit of having a separate, or separable, electrode array 10 and fluidic component 20 is that the fluidic component 20 may be removed following the implantation of the electrode array 10. However, in other scenarios it may be acceptable (or indeed preferable) to keep the fluidic component 20 *in situ* to remain part of the implanted device 100.

The flexible substrate 30 may be 500 µm thick or less, optionally 200 µm thick or less, further optionally 100 µm thick or less, further optionally 50 µm thick or less, further optionally 25 µm thick or less, further optionally 10 µm thick or less and still further optionally 5 µm thick or less. A thin substrate facilitates the creating a small gathered configuration of the medical device 100.

The flexible substrate 30 may be made of a polymeric material, optionally a thermoplastic, and optionally comprising one or more of a poly-urethane, a silicone, a parylene, a polyamide, a polyimide, a cyclic olefin polymer, a cyclic olefin copolymer, a polyacrylate, polyethylene terephthalate and/or an epoxy. Such materials are suitable for implantation in the body, and provide the flexibility to facilitate configuring the device in a gathered configuration that can be actuated into an expanded configuration.

In the illustrated embodiment of Figure 1, the fluidic component 20 comprises a fluidic channel 21 that extends through the substrate 30, with an inlet 22 and an outlet 23. The inlet 22 (and outlet 23) may be embodied, for example, as a tube formed separately and subsequently connected to the fluidic channel 21.

The inlet 22 is for supplying fluid (i.e. liquid or gas) into the fluidic component 20. In general, there may be one or more such inlets 22. Such supply actuates the fluidic component 20. The actuation may be the result of the supply increasing a fluid pressure and/or an amount of a fluid within the fluidic channel 21 of the fluidic component 20. In some arrangements, the supply of fluid may cause an inflation or straightening of the channel 21 within the substrate 30, for example.

In some arrangements, there may be no specific outlet 23, separate to the inlet 22. For example, when using a gas as the actuating fluid, the gas may be supplied to inlet 22 to actuate the device 100, and removal of the supply may subsequently allow the pressure to be released within the channel 21 and gas to exit the channel 21 via the original inlet 22. In other arrangements, the channel 21 may extend from a dedicated inlet (or inlets) 22 to one or more separate outlets 23.

In some arrangements, the fluidic component 20 may have independent fluidic channels 21, each with their own inlets 22 and outlets 23 (if present).

In either case, the route of the channel 21 through the substrate 30 can take different forms. The form of the route may be dictated by the manner in which the device 100 will be arranged into the gathered configuration, and the manner in which it is desired for the device to transition into the expanded configuration. In some arrangements, the channel 21 may have a branching and/or symmetrical structure within the fluidic component 20. Such arrangements can provide an even distribution of the channel 21 throughout the substrate 30, which can be advantageous for even deployment of the device 100. The channel 21 may take the form of a single chamber (e.g. having a 'balloon' or 'pillow' form when inflated), or a series of interconnected chambers of that sort. Larger chambers may also have connecting ties or 'pillars' from one side of the chamber to the other, to help control the inflated shape and resist over-inflation.

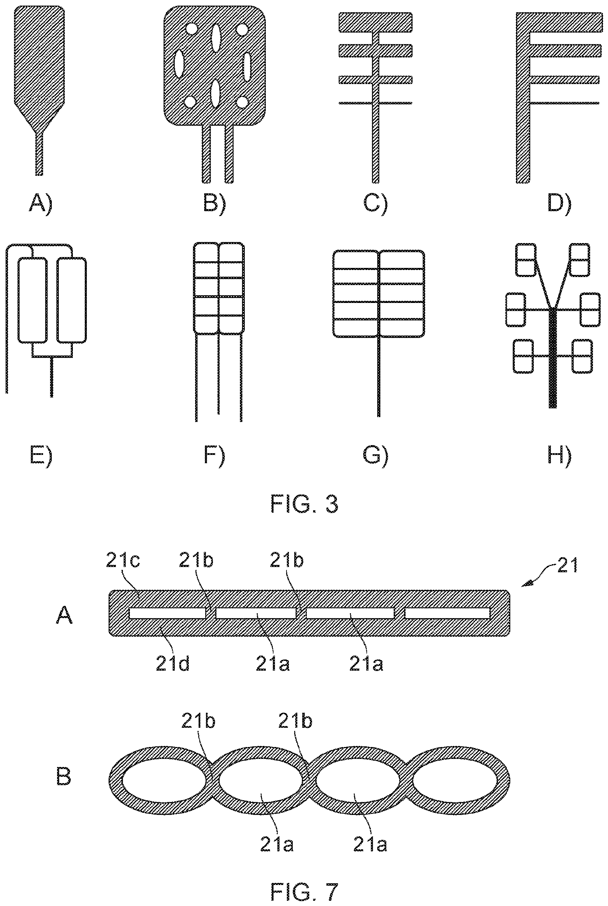

Fig. 3 illustrates various patterns (in plan view) that may be used for the channel 21 of the fluidics component, although other patterns are also possible. In the patterns, black represents the channel 21 and areas of white within the black areas indicate areas where the channel 21 does not extend, such as the ties or 'pillars' mentioned above, or larger areas of the substrate encircled by the channel 21.

As will be observed, the patterns of Figs. 3A, 3C, 3D, 3G and 3H have a single inlet (at the bottom of each pattern), which can function as both inlet and outlet. Fig. 3B has two off-centre lines at the bottom, which could both be simultaneously used as inlets and subsequently simultaneously used as outlets, or could be provided as dedicated inlet and outlet lines. Fig. 3E has a line approaching the pattern from the bottom, and a line leading away from the top - this arrangement provides a natural 'flow-through' arrangement in which e.g. the bottom line can act as an inlet and the top line as the outlet, or vice versa. Fig. 3F has three lines approaching from the bottom; as for Fig. 3B, these could all be used together as inlets or outlets, depending on the need, or could be individually dedicated as inlet or outlet channels. For example, the central line may be the inlet, whilst the outer lines are outlets, or vice versa.

It will also be observed that the patterns of Figs. 3A, 3B, 3C, 3F, 3G and 3H are symmetrical in nature. As mentioned above, this can assist with even unrolling/unfurling. The design of Figs. 3D and 3E are predominantly symmetrical too, other than the arrangement of the single inlet/outlet line of 3D, and the inlet/outlet line returning from the top of the pattern to in Fig. 3E.

Considering the patterns individually, Fig. 3A illustrates a channel 21 forming a single chamber. That chamber is of the 'balloon' or 'pillow' type, containing no ties. The chamber has a typical paddle shape that might correspond to the shape of an SCS electrode array, for example.

Fig. 3B illustrates a channel forming a large chamber, but compared to Fig. 3A the Fig 3B chamber is squared, of the sort of shape that may be useful for cortical sensors (designed for the surface of the brain). The chamber of Fig. 3B also contains ties or 'pillars' that connect the upper and lower sides of the chamber. Those ties are shown as the white circles and ovals. The ties help control how the chamber inflates and reinforce the chamber design.

Fig. 3C illustrates a branching channel design, with branches in both directions (left and right) from a central channel. The branches get thicker towards the top of the pattern (i.e. further from the inlet/outlet line at the bottom of the pattern). Such an arrangement provides less resistance to flow in the thicker branches, and can help encourage fluid to fill the whole pattern as it is introduced, rather than just fill from the end closest to the inlet.

Fig. 3D is a branching design similar to Fig. 3C, but with the inlet line offset to the side, such that the branches extend from that line in one direction (i.e. to the right as depicted).

Fig. 3E is a branching design in which the inlet branches, and then those branches also branch, before the various branches come back together again. This design effectively creates encircled areas of the substrate, bounded by the channel. Although the channel does not pass through those areas, the presence of the channel around those areas means the unfurling of that area is still actuatable.

Fig. 3F is a multiply branching design which creates a network of channels and encircled areas. Fig. 3G is a similar design (although wider) with a different inlet arrangement as already discussed.

Fig. 3H is a branching design in which the initial branches are not interconnected, but which each subsequently branch further to form local networks of channel at different positions within the substrate. Such an approach might be desired, for example, to provide a concentration of the channel (i.e. the networked areas) in regions that will correspond to electrode locations, to ensure those areas are particularly well unfurled.

Although various arrangements have been discussed with respect to Fig. 3, the skilled person will recognise that those are illustrative only, and that other variations and designs are also possible including designs with multiple independent fluidic chambers.

In some arrangements, the fluidic channel 21 may be embedded wholly within the substrate 30, such that the channel 21 is merely defined by the absence of the substrate material within the channel. In other arrangements, the channel may be formed of a different material to the surrounding substrate 30, or may be formed of the same material as the substrate 30 but not embedded directly within that substrate 30. As such, the fluidic channel may be relatively flexible or rigid compared to the substrate, depending on the method of construction. In either case, the fluidic channel may have a maximum uninflated width dimension (i.e. a maximum size across a cross-section through the channel 21 perpendicular to the centreline of the channel 21, before the channel is expanded by pressurisation or being filled with fluid) of 5 mm or less, optionally 3 mm or less, further optionally 1 mm or less, further optionally 500µm or less, further optionally 100µm or less, further optionally 50µm or less, and still further optionally 5µm or less. The fluidic channel may also have a maximum inflated thickness (i.e. a maximum dimension following the expansion of the channel after it is pressurised/ filled with fluid to actuate the fluidic component) of no more than 5 mm, optionally no more than 2 mm, further optionally no more than 1 mm, and still further optionally no more than 500µm.

Figure 2 illustrates how the flexible nature of the medical device 100 can be exploited to assist in its deployment. Because the electrode array 10 and the fluidic component 20 are both flexible, the entire device 100 can be gathered into a configuration that can permit percutaneous deployment. In particular, the flexibility of the electrode array and, preferably, the fluidic component at least in the gathered configuration, can allow the medical device to be rolled without the functionality of the electrode array being affected.

The left hand side of Figure 2A illustrates, in side view, the device 100 rolled along the longitudinal extent of the device 100. That is, the device 100 is rolled along its longest axis. The device 100 can be unrolled to a relatively flat configuration, shown on the right.

Figure 2B shows an alternative arrangement (this time in plan view). On the left, the device 100 is rolled or folded across the width (the shorter direction, within the plane of the electrode array 10 when expanded to a flat configuration) of the device. Again, the device may subsequently be unrolled or unfolded to provide the fully deployed device as shown on the right.

In both cases, the gathered configuration of the device 100 allows for the possibility of the device 100 to be implanted percutaneously. By providing a suitably thin and flexible substrate 30, even a device 100 with a relatively large expanded surface area can be rolled into a relatively narrow configuration that allows for percutaneous deployment with a suitable needle. Preferably, the gathered configuration is such that the maximum width of the device (i.e. in a cross section in the direction of gathering) in that configuration is 1 cm or less, optionally 5 mm or less, further optionally 2 mm or less. It is advantageous for the maximum width to be as small as possible, as this allows for a smaller diameter needle to be used for the percutaneous deployment. As such, it may be advantageous to roll the device 100 in the narrower width dimension of the device 100 as opposed to the longer length dimension, to arrive at a smaller gathered width (as there will be less material to gather).

Although Fig. 2A illustrates an example with a single roll, it may be advantageous to roll, fold or otherwise gather the device from two directions, as shown in Fig. 2B, e.g. from two edges to a centre line. Such an arrangement can allow for a more even deployment, as discussed below. That is, it can allow the two sides to deploy at the same time, thereby avoiding twisting of the device 100 in situ as it is placed.

The method of gathering will be determined by the particular device, but it can e.g. be performed by hand, using a guide or otherwise, or may be automated. The gathering may use a guide component (which may be integral to the device 100, or a separate component) to give additional stiffness/structure to the gathered device 100, to assist with the percutaneous delivery. Such a guide component may take the form of a wire or tubing, or a bio-resorbable shank, either within or around the gathered device 100. That is, the guide component may provide a relatively rigid 'backbone' or support around which the device 100 may be gathered, and which may be subsequently used to help direct the device to its deployment location from within the gathered configuration. Alternatively, or in combination, the guide component may be a sheath or tube which the device is fed into as/after it is gathered, so that the guide component is outside of gathered device. In the case of an internal guide component, that component may or may not be removed once the device 100 is deployed. In the case of an external tube or sheath, the guide component must be withdrawn or retracted relative to the device enough to allow the change to the deployed configuration (although, in some cases this may be possible without any retraction at all, e.g. if internal and external guide components are used in combination).

In use, the device 100 may be gathered as discussed above, and then initially deployed according to methods known in the art. For example, an SCS device may be deployed percutaneously. Alternatively, a brain sensor can be deployed through a burr hole in the cranium. Such a burr hole can be 20 mm or less in diameter, further optionally 10 mm or less, further optionally 5 mm or less, and still further optionally 2 mm or less.

After the initial deployment, fluid may be supplied to inlet 22 to fill and/or pressurise the channel 21. As the channel 21 is filled/pressurised, it is urged into its expanded configuration, and therefore begins to unroll/unfold the fluidic component 20. As such, the transition of the fluidic component 20 from the gathered configuration to the expanded configuration is actuated by supplying fluid to the fluidic channel 21. This transition brings the device into contact with, or into suitable proximity with, the target tissue.

The change in configuration of the fluidic component 20 causes a change in configuration of the associated flexible electrode array 10. In the embodiment of Fig. 1, the electrode array 10 comprises the substrate 30 in which the fluidic component 20 is comprised. As mentioned above, in other arrangements, the fluidic component 20 and the electrode array 10 may be separate, or separable, components that are each independently flexible. In those arrangements, by virtue of the separate/separable components being gathered together, the actuation of the fluidic component still causes the change in configuration of the electrode array 10, even though the electrode array 10 and the fluidic component 20 do not share the same substrate 30, for example.

The fluidic actuation of the device 100 causes the device 100 to expand into a configuration having a greater projected surface area than the expanded configuration. The expanded shape and area of the electrode array varies depending on the application. For example, the electrode array for a brain sensor may be relatively square or circular and have dimensions, for example, up to 100 mm by 100 mm (i.e. a total area of 0.01 m²) or even larger. SCS devices, in contrast, may have similar total areas but are relatively long and thin and may have dimensions up to 30 mm by 300 mm, or larger. In either case, smaller devices may be used for more targeted sensing/stimulation. Moreover, the fluidic component 20 can act as a support to help with the positioning of the expanded electrode array 10. The fluidic component 20 could be, for example filled with a self-curing gel or foam following deployment, to provide ongoing rigidity and support.

Once the device 100 has been deployed and positioned, the fluid provided to the channel 21 may be removed. However, this is not necessary. For example, the fluid may be a saline solution or similar which provides no clinical risk in the unexpected scenario that the fluid somehow escapes from the device 100. Similarly optionally, the fluidic component 20 may itself be removed following the positioning of the electrode 10, provided that the fluidic component 20 and the electrode array 10 are separate or separable. For example, if the fluidic component 20 and the electrode array 10 are entirely separate, the fluidic component 20 may be actuated to cause the change in configuration, thereby unfolding both the fluidic component and the electrode array 10, and following that unfolding the fluidic component 20 may be freely removable.

Following the deployment and positioning of the implant 100, the implanted device 100 may be used in the desired capacity, whether that is a sensor or as a stimulator in the treatment of the patient. Such treatment can include therapy or diagnosis, or may be as part of a method of surgery.

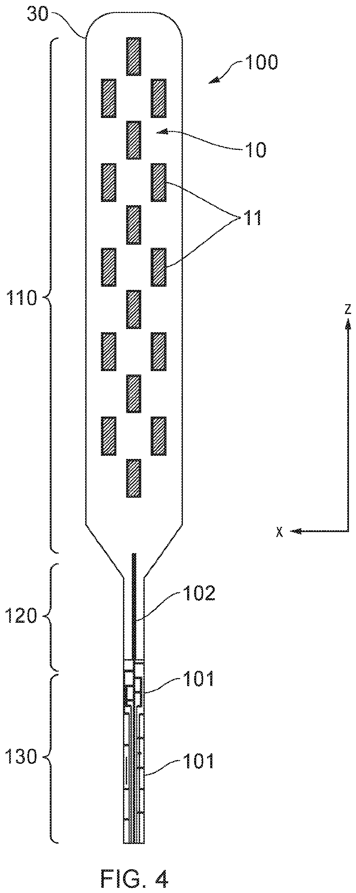

Figure 4 shows a medical device 100 of an embodiment of the present invention in an inflated state. The components of the device 100 visible in Figure 4 are labelled using the same numbering as in Figures 1 and 2. Generally, the electrode array 10 including a plurality of Ti/Au or Pt electrodes 11 can be seen covering the substrate 30 at the distal or functional end 110 of the device.

At the proximal end of the device, a first section 120 provides one or more fluid connectors 102 for fluidic connection for connection of the fluidic component 20 to an external inflation device. The fluid connectors 102 are medical grade polyethylene tubing (although other materials may be used as indicated above) and have an outside diameter of less than about 1mm.

A second, more proximal, section 130 provides one or more electrical connectors 101 for electrical connection of the electrode array 10 to external electronics such as a pulse generator for stimulation or sensors for recording data from the electrodes. The electrical connectors 101 are three copper/polyimide flex cables each with a thickness of about 0.07mm.

In the arrangement in Figure 4, the distal end 110 is the portion of the device 100 whose configuration can be changed from a gathered or compressed arrangement into a larger, deployed arrangement when the fluidic component is actuated. This distal end 110 is generally flexible, whilst the first and second sections 120, 130 at the proximal end of the device may be less flexible or even rigid, thereby allowing for secure connection from the external sources to the fluidic component 20 and the electrode array 10. It will be appreciated that the fluid connectors 102 and the electrical connectors 101 will likely not connect directly to the external sources but may be connector to further elements such as tubing or wires (not shown) which extend away from the medical device 100 and, when the device 100 is deployed within a patient, may extend outside of the patient through a lumen. The first and second sections 120, 130 are also not inflatable and do not change shape or configuration when the fluidic component is actuated.

In particular, the distal end 110 of the device 100, and in particular the electrode array 10, has a bend radius of no more than 2mm in the x-direction as shown in the axes in Figure 4. This means that the device can be readily rolled into a gathered configuration by rolling about the centre line of the device 100 which is parallel to the z direction in the manner shown in, and described above in relation to, Figure 2B, and then deployed from that rolled configuration into the arrangement shown in Figure 4 when the fluidic component is actuated.

In the device 100 shown in Figure 4, the device is significantly less flexible to bending about axes parallel to the x direction shown (perpendicular to the z direction). Thus the device 100 shown in Figure 4 is not suitable for deployment in the manner shown in, and described above in relation to, Figure 2A. This arrangement allows the device 100 to have certain rigid or less flexible components in the distal portion 110, provided that they are aligned along the longitudinal extent of the device 100.

For example, the distal portion 110 of the device 100 may have a support (not shown) which extends along the central longitudinal axis of the device in the z direction. This support can provide support and rigidity to the device 100 which may be needed, for example, to facilitate deployment and/or to ensure the device retains a desired longitudinal configuration when deployed. Despite this rigid or less-flexible support, the distal portion 110 of the device 100 can still be gathered into a compressed configuration by rolling the two sides in to form two coils (as viewed along the z direction) which meet at the central axis.

It will be appreciated that, in alternative embodiments, the device 100 may be more flexible in the z direction shown in Figure 4 and less flexible (or rigid) in the x direction. This would allow for rolling and deployment of the device in the manner shown in, and described above in relation to, Figure 2A. In such a device, rigid or less flexible components in the distal portion 110 could be aligned parallel to the x direction (i.e. transverse to the longitudinal extent of the device 100).

In a variation on such devices 100, the rigid or less flexible components in the distal end 110 may be detachable or removable. For example, a rigid or stiff support may be used which extends along the longitudinal extent of the device 100 during deployment of the device into a patient to prevent the distal end of the device from being squashed or deformed during deployment. This support may then be removed once the device is in the desired position. In these variant devices, once all the rigid or less flexible components have been removed or detached from the distal end, the distal end may be flexible in both the x and z directions and may have similar bend radii in both directions.

In alternative embodiments, the distal end 110 of the device 100 may have no rigid or less flexible components and thus be similarly flexible in both the x and z directions. Such devices may be configured so that deployment by unrolling or unfurling once the device has been deployed is possible in both the x and z directions.

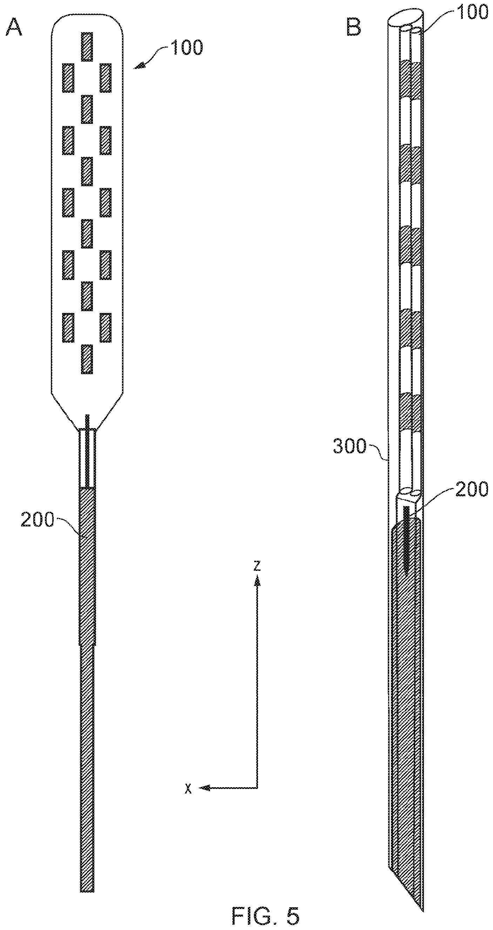

Figure 5 illustrates how a device 100 such as that illustrated in Figure 4 and described above may be packaged for deployment. The device shown in Figure 5 is identical to that shown in Figure 4 and the individual components will not be described again.

Figure 5A shows how a first sheath or connection tubing 200 covers the fluidic and electrical connectors. The first sheath 200 is medical grade polyurethane (although, as above, alternative materials may be used) having an interior diameter of about 1.5mm and a wall thickness of about 0.07mm. The first sheath 200 covers and protects the fluidic and electrical connectors (and the further tubing and/or wires etc. that those connectors are connected to).

Figure 5B shows the device 100 in a rolled configuration (double-rolled about axes parallel to the z direction as described in relation to Figure 4 above), along with the first sheath 200, both contained within a second sheath or deployment tubing 300. The second sheath 300 is medical grade polyurethane (as above, alternative materials may be used) having an interior diameter of about 1.82mm and a wall thickness of about 0.15mm.

It will be appreciated that, in order to fit into the second sheath 300 in a double-roll configuration without being damaged, the distal portion 110 of the device, and thus the fluidic component 20 and the electrode array 10 need to have a bend radius of less than 0.455mm (1.82mm/2 = 0.91mm maximum available diameter space for each roll => 0.91mm/2 = 0.455mm maximum radius for each roll).

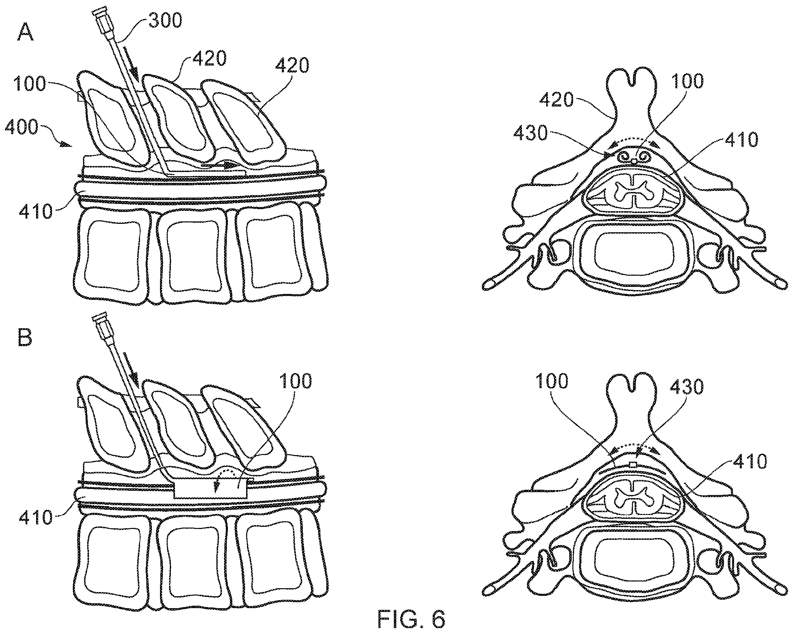

Figure 6 shows the deployment of a medical device 100, such as that shown in Figures 4 and 5, into the spinal cord area 400 of a patient according to an embodiment of the present invention. The device 100 shown in Figure 6 is an SCS device which is designed to lie alongside the spinal cord 410. In each of Figure 6A and 6B, the left hand side drawing is a side view of the patient along a cross-section through the spinal cord, whilst the right hand side drawing is a transverse cross section through the spinal cord at a point where the device is located.

Figure 6A shows how the device 100 is inserted (for example using a sheath 300 or other catheter delivery system) between vertebrae 420 so as to lie substantially parallel to the spinal cord 410. The right hand drawing shows how the device 100 starts to be deployed by fluidic actuation which causes the two rolls to unroll outwards away from the centre line of the device 100.

Figure 6B shows the device 100 in a deployed state in which the device is fully unrolled or unfurled and has a substantially planar configuration, although flexibly conforming to the curvature of the spinal cord 410 so that the electrode array of the device lies adjacent to the spinal cord.

It can be seen, particularly from the right hand drawings in Figures 6A and 6B, that the epidural space 430 available for the device 100 to deploy into is limited in the vertical direction of Figures 6A and 6B (the anterior-posterior (AP) dimension in terms of the patient). In order for the device 100 to deploy into this space, it is advantageous that the device can unroll or unfurl such that its thickness in the vertical direction does not significantly exceed (if at all) the thickness of the device in that direction when the device is in the gathered or compressed configuration that it is initially inserted in. Devices comprised of multiple layers which unfold when deployed would be less suitable (if at all) for deployment in such spaces.

Whilst this limited space is particularly the case in the deployment of spinal cord stimulators and other devices into the spinal cord area 400, similar limitations apply in the deployment of other medical devices, for example to the brain area.

As well as meaning that the space for deployment of the device 100 is limited, the restrictions in this direction also mean that any expansion of the device in this direction (i.e. perpendicular to the direction of the unrolling) needs to be limited and ideally does not substantially exceed (if at all) the thickness of the device in the gathered configuration that it is inserted in. Excessive expansion in the vertical direction can lead to damage to surrounding tissue, obstruction of blood vessels or other complications.

Simple inflation of a fluidic component such as that found in known inflatable devices would typically tend to result in a thin, flat deflated structure with a thickness of, say, 20-500 microns inflating to adopt a bulbous shape, often having a circular or oval cross-section of up to 1cm thickness. This would not be practical in the implementations discussed above in relation to Figure 6.

It has been suggested that the inflation thickness of devices could be controlled by limiting the amount or pressure of the inflation fluid injected into the device during deployment. However, in practice, a significant pressure build-up inside the fluidic components of the device is needed to initiate the deployment from the compressed configuration to the deployed configuration. As the force needed to cause expansion of the fluidic component (and therefore the device as a whole) in the vertical direction is typically less that the force needed to cause the device to deploy, the inflation necessary for deployment in such devices will inevitably lead to undesirable vertical expansion.

The devices 100 of certain embodiments of the present invention are designed so as to limit the expansion of the device in the vertical direction (i.e. a direction perpendicular to the direction of deployment of the device and/or a direction perpendicular to the substantially planar arrangement of the device in its deployed configuration). In certain configurations, the device is limited so that the thickness of the device in the vertical direction in the deployed configuration (and preferably at all stages during deployment) is never greater than the dimensions of the device in that same direction in the gathered configuration prior to deployment. In certain applications, this may be no more than a few millimetres (e.g. 2, 3 or 5mm).

A variety of arrangements of the device 100 and/or the fluidic component 20 may be used to achieve this. Two or more of the arrangements described further below may, of course, be combined in a particular embodiment.

In certain embodiments, the limitation on vertical expansion is achieved by incorporating a stiff (or alternatively inelastic or minimally elastic) material into one or more layers above and/or below the fluidic channel 21. This stiff material resists deformation and therefore restricts expansion in the vertical direction. Incorporation of such stiff material needs to also take account of the requirements for the overall flexibility of the device for the deployment process. This could, for example, be achieved by providing strips of stiff material with regions of flexible material between them, the strips being oriented perpendicular to the direction of unrolling or unfurling of the device during deployment, such that the flexible material ensures that the device as a whole is still sufficiently flexible to deploy, while the stiff strips prevent or reduce the vertical expansion by increasing the force needed to cause such expansion.

In a variant of the above, a material could be used to form a layer in the device above and/or below the fluidic channel 21 which has anisotropic properties, such that it is flexible in the direction of rolling/unrolling, but stiff or inelastic in the perpendicular (e.g. longitudinal) direction.

In certain embodiments, the fluidic channel 21 itself is configured to limit vertical expansion. For example, the fluidic channel 21 may have a cross-section such as that shown in Figure 7. Figure 7A shows a schematic cross-section through the fluidic channel 21 when the device 100 is in an uninflated (i.e. compressed/gathered) state (for convenience, the channel is shown planar, although in reality it is likely to be rolled/bent in that state). The channel 21 is divided into a plurality of parallel sub-channels 21a by a plurality of ties or posts 21b that physically bond the top and bottom layers 21c, 21d and thereby constrain vertical expansion of the fluidic channel. The ties or posts 21b can be manufactured as part of the channel itself, or may be formed by bonds or welds between the top and bottom layers 21c, 21d. The ties or posts 21b may be spot joins, with a plurality of such joins distributed along the channel, or may be contiguous along all or part of the channel 21.

As shown in Figure 7B, when the device is inflated, the expansion of the fluidic channel 21 in the vertical direction is restrained or restricted by the ties or posts 21b and therefore, whilst the individual sub-channels 21a can expand vertically, the overall expansion of the device can be controlled and limited.

In a similar fashion, if the fluidic channel 21 is sufficiently small in cross section, then vertical expansion may be prevented or restricted as for the individual sub-channels 21a shown in Figure 7. Thus an overall design of the fluidic channel 21 which is small in cross-section may be provided. A plurality of such channels may be arranged in parallel to each other and be joined at either end.

In other embodiments, there are multiple fluidic chambers defined along the fluidic channel 21 which are arranged to fill sequentially on inflation of the device. Pressure-controlled valves are arranged between each of the chambers such that a chamber will inflate to a predetermined pressure limit before the valve connecting to the next chamber is forced open. This process could be repeated throughout the device. The vertical expansion of the device can then be limited by the design of the geometry of the chambers and the pressure limits set by the valves.

Having discussed the configuration and use of the device 100, the following sections consider options for fabrication of such a device 100. The discussion presents two options for how the electrode array 10 may be formed, and then three separate options for how the array may be integrated with the fluidic component 20. Although these protocols refer to specific manufacturing techniques, the skilled person will understand that other techniques may be substitutable to produce the devices, depending on the desired materials etc. Such processes include for example photolithography process, casted elastomer processes, digital manufacturing processes (controlled extrusion, additive manufacture).

### Fabrication of electrode array

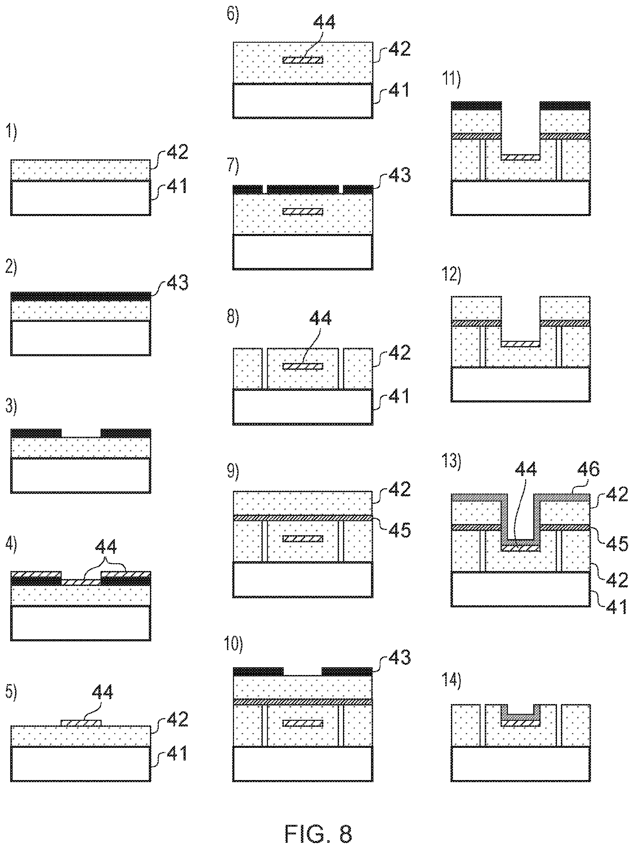

As shown in Fig. 8, step 1, a clean and rigid substrate 41 (of any suitable material, such as glass or silicon wafer) can used for the deposition of a thin layer of flexible material 42 (which will ultimately form part of the electrode array 10). Suitable flexible materials include, but are not limited to, parylene, silicones, polyurethanes, other thermoplastic polymers, etc. Prior to the deposition of the flexible material 42, a release layer might be used to minimise adhesion of the flexible material film 42 with the rigid support 41 and ease release of the final structure.

The thin layer of flexible material 42 can then serve (Fig. 8, steps 2 - 5) as a base onto which electrodes and conductive lines are patterned. The patterning may use, but is not limited to using, metals such as gold, iridium and/or platinum. The patterning can be achieved through lift-off techniques that will be familiar to those skilled in the art. Briefly, a photoresist layer 43 can be spin coated (Fig. 8, step 2), soft baked and exposed (typically by UV light) using a contact aligner. The exposed photoresist 43 can then be developed (Fig. 8, step 3) in the appropriate developer. A layer of an adhesion promoter metal typically chromium or titanium) can then be deposited. That layer may be 5 to 10 nm thick, for example. That can be followed (Fig. 8, step 4) by the deposition of a relatively thick layer 44 of the electrode/conductive material - e.g. gold or platinum. That layer 44 may be 100 nm, for example, or thicker. Multilayer deposition of different metals can also be performed. The final metal patterns are obtained (Fig. 8, step 5) through lift-off of the photoresist 43 in a suitable photoresist removal medium (aqueous solution or solvent/solvent mixture).

Although not illustrated, pattering of the electrodes and conductive lines could instead be performed via wet or dry etching of a metal layer. Another possible metal pattering technique is laser ablation of a conformal metal foil adhering to the base thin plastic layer.

Following the creation of the patterned electrode array, the microfabrication of the device 100 can continue with the deposition of a second film of the flexible material 42 (Fig. 8, step 6). This layer serves as a passivation layer for the electrodes 11.

Optionally, an adhesion promoter might be used to improve adhesion between the base layer and the passivation layer of the flexible material 42. By way of example, a typical adhesion promoter for parylene is A-174 (Methacryloxypropyl trimethoxysilane). Alternatively, roughening of the surface of the base plastic layer can also improve adhesion of the passivation layer.

Next (Fig. 8, step 7) another photoresist 43 can be spin coated, exposed, and developed using appropriate developer, followed by e.g. reactive ion etching (Fig. 8, step 8) to define the outline of the device. Residual photoresist after the dry etching step can removed using the appropriate solvent. Alternatively, the device outline can be defined through laser ablation of the two thin plastic layers, for example.

A third sacrificial layer of flexible material 42 can then be deposited (Fig. 8, step 9) on this structure. An anti-adhesion layer 45, e.g. a 2% v/v soap solution, can be spin coated in between the second and third layer of flexible material to minimize adhesion.

The fabrication can then continue (Fig. 8, step 10) with the deposition of a layer of photoresist 44 to define the electrode area. The photoresist 44 can then be exposed and developed, followed by dry etching of the sacrificial and passivation layers of flexible material 42 (Fig. 8, step 11).

This step is then followed (Fig. 8, step 12) by a lift-off of the remaining photoresist 43 in the suitable photoresist removal medium (aqueous solution or solvent/solvent mixture).

After dry etching, an aqueous dispersion of a conducting polymer 46 can be spin coated (Fig. 8, step 13) on the substrate. The conducting polymer 46 may be poly(3,4-ethylenedioxythiophene)- poly(styrenesulfonate) (PEDOT:PSS), for example, and may contain additives (such as 5 vol% of ethylene glycol, 0.1 vol% dodecyl benzene sulfonic acid, and 1 wt% of (3-glycidyloxypropyl) trimethoxysilane (GOPS)). Multiple depositions can be used to control the thickness of the conducting polymer. An, e.g. 1 min, step of soft baking (e.g. at 110°C for the PEDOT:PSS system mentioned above) may be used in between each deposition.

Finally (Fig. 8, step 14), the sacrificial third layer of flexible material 42 is removed to complete the patterning of conductive polymer and hard baked (e.g. at 140 °C for 1 hour).

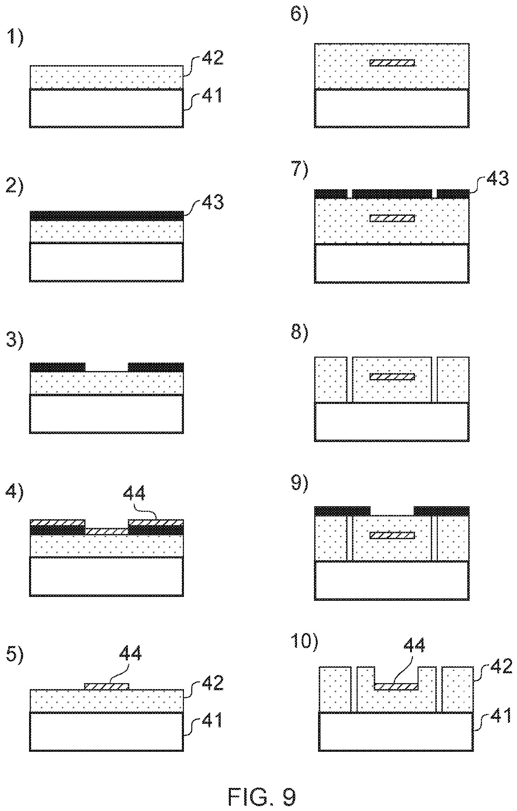

The above described protocol includes the patterning using an organic material (i.e. the conductive polymer, such as PEDOT:PSS). However, the device 100 can be manufactured without the organic layer 46. In that case the workflow is slightly different, and is illustrated in Fig. 9 are as discussed in connection with Fig. 8 above. Then, following the creation of the etched device outline, a layer of photoresist 43 can be applied to define the electrode area (Fig. 9, step 9), to allow the electrode area to be etched (Fig. 9, step 10) and the remaining photoresist 43 to be removed.

Alternatively, other methods of pattering with organic material such as PEDOT:PSS can be used, such as via (1) dry etching, (2) dip coating, (3) photopolymerization or (4) electropolymerized on the electrode surface

### Integration of electrode array and microfluidics

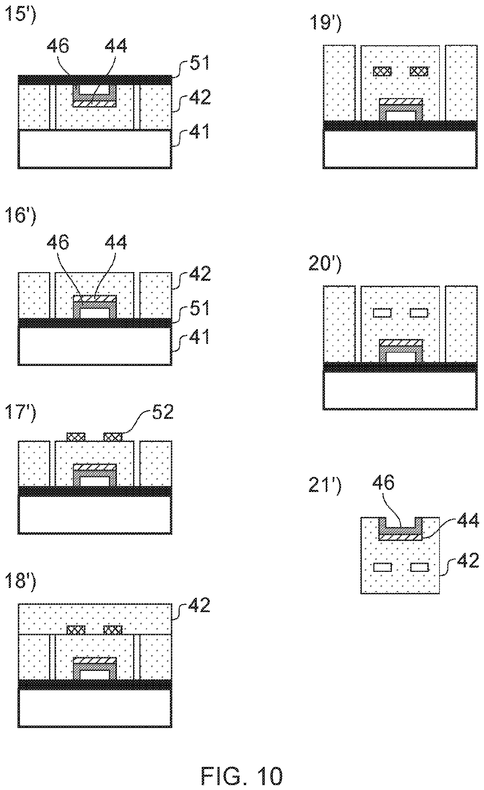

A first strategy for integrating the electrode array created by the methods discussed in connection with Figs. 8 and 9 is illustrated in Fig. 10, steps 15' to step 21'. The device can be coated with a water-soluble sacrificial layer 51 (Fig. 10, step 15'). This could be an aqueous PVA (poly-vinyl alcohol) solution, for example. The device can then be released from the rigid substrate 41 and reposition on the same or different rigid substrate 41 having now the sacrificial layer 51 facing down onto the rigid substrate 41 (Fig. 10, step 16'). A removable patterning material 52 such as glycerol can be used to define the microfluidic structure (Fig. 10, step 17'), prior to deposition of another layer of flexible material (Fig. 10, step 18') to seal the microfluidics. The outline of the microfluidic is then defined (Fig. 10, step 19'). The removable patterning material is then removed (Fig. 10, step 20') and a tubing is positioned and fixed at the inlet of the microfluidics. The final structure is then released (Fig. 10, step 21').

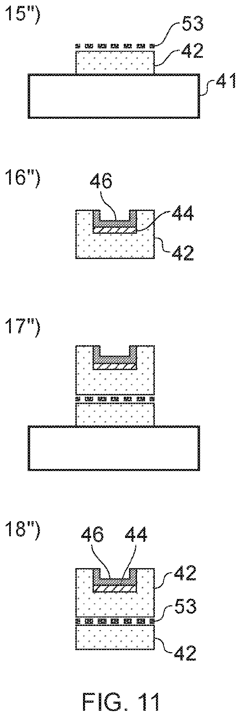

A second strategy for integrating the electrode array created by the methods discussed in connection with Figs. 8 and 9 is illustrated in Fig. 11, steps 15" to step 18". The microfluidic structure is designed using a CAD software. A thin and flexible double-sided tape 53 is laser cut (e.g. CO2 laser cut) to define the microfluidic structure (Fig. 11, step 15"). One side of the tape can be permanently bonded to a pristine layer of the flexible material 42 (which may be held on a support 41). Next, the bioelectric device (e.g. as created according to the methods described in connection with Figs. 8 and 9) can be released from its own substrate 41 (Fig. 11, step 16"), and then aligned and bonded (Fig. 11, step 17") to the other side of the tape in order to seal the microfluidics. A tubing can then be positioned and fixed at the inlet of the microfluidics. The final structure can then be released (Fig. 11, step 18").

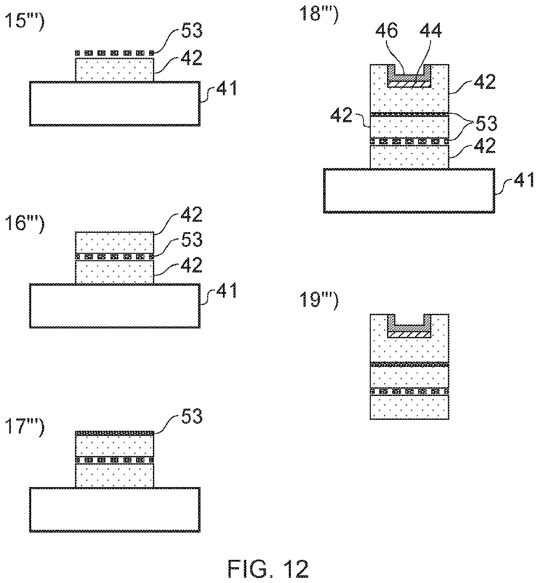

A third strategy for integrating the electrode array created by the methods discussed in connection with Figs. 8 and 9 is illustrated in Fig. 12, steps 15‴ to step 19‴. As in the second strategy, the microfluidic structure can be designed using a CAD software, and a thin and flexible double-sided tape 53 is laser cut (e.g. CO2 laser cut) to define the microfluidic structure (Fig. 12, step 15‴). One side of the tape can be permanently bonded to a pristine layer of the flexible material 42 (which may be held on a support 41). The microfluidic device can then be realised (Fig. 12, step 16‴) by positioning a further layer of the flexible material 42 on the other side of the laser cut double sided tape 53. An additional layer of the double-sided tape can be placed on the top side of the microfluidics (Fig. 12, step 17‴), followed by alignment and bonding of the bioelectric device (Fig. 12, step 18‴). A tubing can be positioned and fixed at the inlet of the microfluidics. The final structure can then be released (Fig. 12, step 20‴).

As variations on the second and third strategies, instead of using double-sided tape, a different bonding strategy may be used, such as printing/stamping of viscous adhesive, or laser welding of plastic for example.

The forgoing description is exemplary in nature only, and the skilled person will understand that changes and variations on the disclosed embodiments are possible within the scope of the claims. The claims define the invention.

## Claims

1. A medical device (100), comprising:
a flexible electrode array (10) having a bend radius of no more than about 2mm; and
a fluidic component (20), wherein the fluidic component is fluidically actuatable to cause the fluidic component to change configuration;
wherein the fluidic component and the flexible electrode array are configured such that a change in configuration of the fluidic component causes a change in configuration of the flexible electrode array.

2. The medical device (100) of claim 1 wherein the device has a proximal section (120, 130) and a distal section (110), the flexible electrode array (10) and the fluidic component (20) being arranged in the distal section, and further wherein the distal section has a bend radius of no more than about 2mm in a first direction,
optionally wherein the distal section (110) has a bend radius in a second direction which is orthogonal to said first direction which is more than the bend radius in the first direction,
optionally wherein the medical device is elongate and the first direction is substantially perpendicular to the longitudinal axis of the device.

3. The medical device (100) of any one of the preceding claims wherein the device has a removable support element.

4. The medical device (100) of claim 1, wherein:
the device has a proximal section (120, 130) and a distal section (110), the flexible electrode array (10) and the fluidic component (20) being arranged in the distal section, and further wherein the distal section has a bend radius of no more than about 2mm in a first direction,
the distal section (110) has a bend radius in a second direction which is orthogonal to said first direction which is more than the bend radius in the first direction,
the device has a removable support element, and
when the removable support element is removed, the distal section (110) has a bend radius of no more than about 2mm in each of the first and section directions.

5. The medical device (100) according to any one of the preceding claims wherein the flexible electrode array (10) and the fluidic component (20) are arranged such that a change in configuration of the fluidic component causes the flexible electrode array (10) to transition between a compressed configuration and an expanded configuration having a greater projected surface area than the compressed configuration,
optionally wherein, in the compressed configuration the flexible electrode array (10) and, optionally, the fluidic component (20), is rolled,
optionally wherein, in the compressed configuration, the flexible electrode array (10) is substantially cylindrical,
optionally wherein the transition between the compressed configuration and the expanded configuration includes unrolling of the flexible electrode array (10), optionally wherein the unrolling is about an axis substantially perpendicular to a direction in which the device has a bend radius of no more than about 2mm.
optionally wherein, in the expanded configuration, the flexible electrode array (10) is substantially planar, and/or
optionally wherein, in the expanded configuration, the medical device (100) has a thickness of no more than 5mm, optionally no more than 3mm, optionally no more than 2mm.

6. The medical device (100) of claim 5 wherein the medical device is arranged to limit expansion in the thickness of the device during changes in configuration, by virtue of one or more of the following:
the medical device further comprises a constraining layer which is arranged substantially parallel to the fluidic component (20) and includes one or more portions of inelastic material which are arranged to prevent or limit expansion of the fluidic component in the thickness direction of the device during changes in configuration, or
the medical device further comprises a constraining layer which is arranged substantially parallel to the fluidic component (20) and includes a plurality of strips of stiff material which are arranged substantially parallel to each other and wherein the parts of the constraining layer between said strips are more flexible than said strips, optionally wherein the portions of stiff material are arranged so as not to impede the change of configuration in directions other than the thickness direction.

7. The medical device (100) of claim 5 wherein the medical device is arranged to limit expansion in the thickness of the device during changes in configuration, by virtue of the fluidic component comprising a fluidic channel (21) extending within the fluidic component and the fluidic component further comprising at least one tie (21b) which joins opposing sides (21c, 21d) of the fluidic channel so as to prevent or limit expansion of the fluidic channel in the thickness direction of the device during changes in configuration.

8. The medical device (100) of any one of claims 6 to 7 wherein the fluidic component includes a plurality of independently inflatable chambers wherein the chambers are sized so as to prevent or limit expansion of the fluidic channel in the thickness direction of the device during changes in configuration, optionally wherein the fluidic component further includes a pressure valve arranged fluidically between a first of said independently inflatable chambers and a second of said independently inflatable chambers, said pressure valve being configured such that fluid will not pass from the first chamber to the second chamber until a predetermined fluid pressure is reached in the first chamber.

9. The medical device (100) according to claim 2, or any claim dependent on claim 2, further comprising:
a fluidic connector (102) in fluid communication with the fluidic component (20) and an electrical connector (101) in electrical contact with the electrode array (10), said connectors being provided in the proximal section (120, 130) of the device for connection of the fluidic component and the electrode array to external devices, optionally wherein
the distal section (110) of the device is more flexible than the proximal section (120, 130), and/or
the distal section (110) comprises at least 90% of the volume of the device.

10. The medical device (100) according to claim 9 further comprising a conductive connector connecting the electrode array (10) to the electrical connector (101) and a first sheath (200) which surrounds the conductive connector, optionally further comprising a fluid channel connecting the fluidic component (20) to the fluidic connector (102), wherein the first sheath (200) also surrounds the fluid channel.

11. The medical device (100) according to claim 10 further comprising a second, removable sheath (300) surrounding the flexible electrode array (10), the fluidic component (20), and the first sheath (200), optionally wherein:
the flexible electrode array (10) and the fluidic component (20) are arranged in a compressed configuration within the second sheath (300), and the device is arranged such that actuation of the fluidic component after removal of the second sheath causes the fluidic component and the flexible electrode array to change to an expanded configuration having a greater projected surface area than the compressed configuration; and/or
the internal diameter of the second sheath (300) is 1 cm or less, optionally 5 mm or less, further optionally 2 mm or less.

12. The medical device (100) according to any one of the preceding claims wherein the fluidic component (20) and the flexible electrode array (10) are separate or separable.

13. The medical device (100) according to any one of the preceding claims wherein the medical device includes one or more components which are imageable by X-ray.

14. The medical device (100) according to any previous claim, wherein the flexible electrode array (10) comprises electrodes (11) provided on a flexible substrate (30).

15. The medical device (100) according to any previous claim, wherein the fluidic component comprises a fluidic channel (21) which has:
a maximum uninflated width dimension of 5 mm or less, optionally 3 mm or less, further optionally 1 mm or less, further optionally 500µm or less, further optionally 100µm or less, further optionally 50µm or less and still further optionally 5µm or less; and/or
a maximum inflated thickness of no more than 5 mm, optionally no more than 2 mm, further optionally no more than 1 mm, and still further optionally no more than 500µm.

## Patentansprüche

1. Medizinische Vorrichtung (100), die umfasst:
eine flexible Elektrodenanordnung (10) mit einem Biegeradius von nicht mehr als etwa 2 mm; und
eine fluidische Komponente (20), wobei die fluidische Komponente fluidisch betätigbar ist, um die fluidische Komponente zu veranlassen, ihre Konfiguration zu ändern;
wobei die fluidische Komponente und die flexible Elektrodenanordnung so konfiguriert sind, dass eine Änderung der Konfiguration der fluidischen Komponente eine Änderung der Konfiguration der flexiblen Elektrodenanordnung bewirkt.

2. Medizinische Vorrichtung (100) nach Anspruch 1, wobei die Vorrichtung einen proximalen Abschnitt (120, 130) und einen distalen Abschnitt (110) aufweist, wobei die flexible Elektrodenanordnung (10) und die fluidische Komponente (20) in dem distalen Abschnitt ausgelegt sind, und ferner wobei der distale Abschnitt einen Biegeradius von nicht mehr als etwa 2 mm in einer ersten Richtung aufweist,
wobei optional der distale Abschnitt (110) einen Biegeradius in einer zweiten Richtung aufweist, die orthogonal zu der ersten Richtung ist, der größer als der Biegeradius in der ersten Richtung ist,
wobei optional die medizinische Vorrichtung länglich ist und die erste Richtung im Wesentlichen senkrecht zur Längsachse der Vorrichtung verläuft.

3. Medizinische Vorrichtung (100) nach einem der vorhergehenden Ansprüche, wobei die Vorrichtung ein abnehmbares Stützelement aufweist.

4. Medizinische Vorrichtung (100) nach Anspruch 1, wobei:
die Vorrichtung einen proximalen Abschnitt (120, 130) und einen distalen Abschnitt (110) aufweist, wobei die flexible Elektrodenanordnung (10) und die fluidische Komponente (20) in dem distalen Abschnitt ausgelegt sind, und ferner wobei der distale Abschnitt einen Biegeradius von nicht mehr als etwa 2 mm in einer ersten Richtung aufweist,
der distale Abschnitt (110) einen Biegeradius in einer zweiten Richtung aufweist, die orthogonal zu der ersten Richtung ist, der größer als der Biegeradius in der ersten Richtung ist,
die Vorrichtung ein abnehmbares Stützelement aufweist, und
wenn das abnehmbare Stützelement entfernt wird, der distale Abschnitt (110) einen Biegeradius von nicht mehr als etwa 2 mm in jeder der ersten und zweiten Richtung aufweist.

5. Medizinische Vorrichtung (100) nach einem der vorhergehenden Ansprüche, wobei die flexible Elektrodenanordnung (10) und die fluidische Komponente (20) so ausgelegt sind, dass eine Änderung der Konfiguration der fluidischen Komponente die flexible Elektrodenanordnung (10) veranlasst, zwischen einer komprimierten Konfiguration und einer erweiterten Konfiguration mit einer größeren projizierten Oberfläche als die komprimierte Konfiguration überzugehen,
wobei optional in der komprimierten Konfiguration die flexible Elektrodenanordnung (10) und optional die fluidische Komponente (20) gerollt wird,
wobei optional in der komprimierten Konfiguration die flexible Elektrodenanordnung (10) im Wesentlichen zylindrisch ist,
wobei optional der Übergang zwischen der komprimierten Konfiguration und der erweiterten Konfiguration das Abrollen des flexiblen Elektrodenarrays (10) enthält, optional wobei das Abrollen um eine Achse erfolgt, die im Wesentlichen senkrecht zu einer Richtung verläuft, in der die Vorrichtung einen Biegeradius von nicht mehr als etwa 2 mm aufweist,
wobei optional in der erweiterten Konfiguration die flexible Elektrodenanordnung (10) im Wesentlichen planar ist, und/oder
wobei optional, in der erweiterten Konfiguration, die medizinische Vorrichtung (100) eine Dicke von nicht mehr als 5 mm, optional nicht mehr als 3 mm, optional nicht mehr als 2 mm aufweist.

6. Medizinische Vorrichtung (100) nach Anspruch 5, wobei die medizinische Vorrichtung so ausgelegt ist, dass sie die Ausdehnung der Dicke der Vorrichtung während Änderungen der Konfiguration durch eines oder mehrere der Folgenden begrenzt:
die medizinische Vorrichtung umfasst ferner eine Begrenzungsschicht, die im Wesentlichen parallel zur fluidischen Komponente (20) ausgelegt ist und enthält einen oder mehrere Abschnitte aus unelastischem Material, die so ausgelegt sind, dass sie die Ausdehnung der fluidischen Komponente in Richtung der Dicke der Vorrichtung bei Änderungen der Konfiguration verhindern oder begrenzen, oder
die medizinische Vorrichtung umfasst ferner eine Begrenzungsschicht, die im Wesentlichen parallel zur fluidischen Komponente (20) ausgelegt ist und enthält eine Vielzahl von Streifen aus steifem Material, die im Wesentlichen parallel zueinander ausgelegt sind, wobei die Teile der Begrenzungsschicht zwischen den Streifen flexibler sind als die Streifen, optional wobei die Abschnitte aus steifem Material so ausgelegt sind, dass sie die Änderung der Konfiguration in anderen Richtungen als der Dickenrichtung nicht behindern.

7. Medizinische Vorrichtung (100) nach Anspruch 5, wobei die medizinische Vorrichtung so ausgelegt ist, dass sie die Ausdehnung der Dicke der Vorrichtung während Änderungen der Konfiguration durch die fluidische Komponente begrenzt, die einen sich innerhalb der fluidischen Komponente erstreckenden fluidischen Kanal (21) umfasst und wobei die fluidische Komponente ferner mindestens eine Verbindung (21b) umfasst, die gegenüberliegende Seiten (21c, 21d) des fluidischen Kanals miteinander verbindet, um eine Ausdehnung des Fluidikkanals in Richtung der Dicke der Vorrichtung während Änderungen der Konfiguration zu verhindern oder zu begrenzen.

8. Medizinische Vorrichtung (100) nach einem der Ansprüche 6 bis 7, wobei die fluidische Komponente eine Vielzahl von unabhängig voneinander aufblasbaren Kammern enthält, wobei die Kammern so bemessen sind, dass sie eine Ausdehnung des Fluidikkanals in Richtung der Dicke der Vorrichtung während Änderungen der Konfiguration verhindern oder begrenzen, optional wobei die fluidische Komponente ferner ein Druckventil enthält, das fluidisch zwischen einer ersten der unabhängig voneinander aufblasbaren Kammern und einer zweiten der unabhängig voneinander aufblasbaren Kammern ausgelegt ist, wobei das Druckventil so konfiguriert ist, dass das Fluid erst dann von der ersten Kammer in die zweite Kammer fließt, wenn ein vorbestimmter Fluiddruck in der ersten Kammer erreicht ist.

9. Medizinische Vorrichtung (100) nach Anspruch 2 oder einem von Anspruch 2 abhängigen Anspruch, die ferner umfasst:
einen fluidischen Verbinder (102), der in Fluidverbindung mit der fluidischen Komponente (20) steht, und einen elektrischen Verbinder (101), der in elektrischem Kontakt mit der Elektrodenanordnung (10) steht, wobei die Verbinder im proximalen Abschnitt (120, 130) der Vorrichtung zur Verbindung der fluidischen Komponente und der Elektrodenanordnung mit externen Vorrichtungen vorgesehen sind, wobei optional
der distale Abschnitt (110) der Vorrichtung flexibler ist als der proximale Abschnitt (120, 130), und/oder
der distale Abschnitt (110) mindestens 90% des Volumens der Vorrichtung umfasst.

10. Medizinische Vorrichtung (100) nach Anspruch 9, die ferner einen leitenden Anschluss umfasst, der die Elektrodenanordnung (10) mit dem elektrischen Anschluss (101) verbindet, und eine erste Hülle (200) umfasst, die den leitenden Anschluss umgibt, und die optional ferner einen Fluidkanal umfasst, der die fluidische Komponente (20) mit dem fluidischen Anschluss (102) verbindet, wobei die erste Hülle (200) auch den Fluidkanal umgibt.

11. Medizinische Vorrichtung (100) nach Anspruch 10, die ferner eine zweite, abnehmbare Hülle (300) umfasst, welche die flexible Elektrodenanordnung (10), die fluidische Komponente (20) und die erste Hülle (200) umgibt, wobei optional:
die flexible Elektrodenanordnung (10) und die fluidische Komponente (20) in einer komprimierten Konfiguration innerhalb der zweiten Hülle (300) ausgelegt sind, und die Vorrichtung so ausgelegt ist, dass die Betätigung der fluidischen Komponente nach dem Entfernen der zweiten Hülle bewirkt, dass die fluidische Komponente und die flexible Elektrodenanordnung in eine erweiterte Konfiguration mit einem größeren projizierten Oberflächenbereich als die komprimierte Konfiguration übergehen; und/oder
der Innendurchmesser der zweiten Hülle (300) 1 cm oder weniger, optional 5 mm oder weniger, ferner optional 2 mm oder weniger beträgt.

12. Medizinische Vorrichtung (100) nach einem der vorhergehenden Ansprüche, wobei die fluidische Komponente (20) und die flexible Elektrodenanordnung (10) getrennt oder trennbar sind.

13. Medizinische Vorrichtung (100) nach einem der vorhergehenden Ansprüche, wobei die medizinische Vorrichtung eine oder mehrere Komponenten enthält, die durch Röntgenstrahlen abbildbar sind.

14. Medizinische Vorrichtung (100) nach einem der vorhergehenden Ansprüche, wobei die flexible Elektrodenanordnung (10) Elektroden (11) umfasst, die auf einem flexiblen Substrat (30) vorgesehen sind.

15. Medizinische Vorrichtung (100) nach einem der vorhergehenden Ansprüche, wobei die fluidische Komponente einen fluidischen Kanal (21) umfasst, der Folgendes aufweist:
eine maximale unaufgeblasene Breitenabmessung von 5 mm oder weniger, optional 3 mm oder weniger, ferner optional 1 mm oder weniger, ferner optional 500 µm oder weniger, ferner optional 100 µm oder weniger, ferner optional 50 µm oder weniger und darüber hinaus optional 5 µm oder weniger; und/oder
eine maximale aufgeblasene Dicke von nicht mehr als 5 mm, optional nicht mehr als 2 mm, ferner optional nicht mehr als 1 mm und darüber hinaus optional nicht mehr als 500 µm.

## Revendications

1. Dispositif médical (100), comprenant :
un réseau d'électrodes flexibles (10) ayant un rayon de courbure ne dépassant pas environ 2 mm ; et
un composant fluidique (20), le composant fluidique étant fluidiquement actionnable pour amener le composant fluidique à changer de configuration ;
le composant fluidique et le réseau d'électrodes flexibles étant configurés de telle sorte qu'un changement de configuration du composant fluidique provoque un changement de configuration du réseau d'électrodes flexibles.

2. Dispositif médical (100) selon la revendication 1, dans lequel le dispositif a une section proximale (120, 130) et une section distale (110), dans lequel le réseau d'électrodes flexibles (10) et le composant fluidique (20) sont disposés dans la section distale, et en outre dans lequel la section distale a un rayon de courbure ne dépassant pas environ 2 mm dans une première direction,
éventuellement, dans lequel la section distale (110) a un rayon de courbure dans une seconde direction qui est orthogonale à ladite première direction qui est supérieur au rayon de courbure dans la première direction,
éventuellement, le dispositif médical est allongé et la première direction est sensiblement perpendiculaire à l'axe longitudinal du dispositif.

3. Dispositif médical (100) selon l'une quelconque des revendications précédentes, dans lequel le dispositif comporte un élément de support amovible.

4. Dispositif médical (100) selon la revendication 1, dans lequel :
le dispositif comporte une section proximale (120, 130) et une section distale (110), le réseau d'électrodes flexibles (10) et le composant fluidique (20) étant disposés dans la section distale, et la section distale ayant en outre un rayon de courbure ne dépassant pas environ 2 mm dans une première direction,
la section distale (110) a un rayon de courbure dans une seconde direction qui est orthogonale à ladite première direction qui est supérieur au rayon de courbure dans la première direction,
le dispositif comporte un élément de support amovible, et
lorsque l'élément de support amovible est retiré, la section distale (110) présente un rayon de courbure ne dépassant pas environ 2 mm dans chacune des première et seconde directions.

5. Dispositif médical (100) selon l'une quelconque des revendications précédentes, dans lequel le réseau d'électrodes flexibles (10) et le composant fluidique (20) sont disposés de telle sorte qu'un changement de configuration du composant fluidique amène le réseau d'électrodes flexibles (10) à effectuer une transition entre une configuration compressée et une configuration déployée ayant une surface projetée plus grande que la configuration compressée,
éventuellement, dans la configuration compressée, le réseau d'électrodes flexibles (10) et, éventuellement, le composant fluidique (20), sont roulés,
éventuellement, dans la configuration compressée, le réseau d'électrodes flexibles (10) est sensiblement cylindrique,
éventuellement, la transition entre la configuration compressée et la configuration déployée comprend le déroulement du réseau d'électrodes flexibles (10), éventuellement dans lequel le déroulement s'effectue autour d'un axe sensiblement perpendiculaire à une direction dans laquelle le dispositif a un rayon de courbure ne dépassant pas environ 2 mm,
éventuellement, dans la configuration déployée, le réseau d'électrodes flexibles (10) est sensiblement plan, et/ou
éventuellement, dans la configuration déployée, le dispositif médical (100) a une épaisseur ne dépassant pas 5 mm, éventuellement ne dépassant pas 3 mm, éventuellement ne dépassant pas 2 mm.

6. Dispositif médical (100) selon la revendication 5, dans lequel le dispositif médical est agencé pour limiter l'expansion dans l'épaisseur du dispositif lors de changements de configuration, en vertu d'un ou plusieurs des éléments suivants :
le dispositif médical comprend en outre une couche de contrainte qui est disposée sensiblement parallèlement au composant fluidique (20) et comprend une ou plusieurs parties de matériau inélastique qui sont disposées pour empêcher ou limiter l'expansion du composant fluidique dans la direction de l'épaisseur du dispositif lors des changements de configuration, ou
le dispositif médical comprend en outre une couche de contrainte qui est disposée sensiblement parallèlement au composant fluidique (20) et comprend une pluralité de bandes de matériau rigide qui sont disposées sensiblement parallèlement les unes aux autres et dans lequel les parties de la couche de contrainte entre lesdites bandes sont plus flexibles que lesdites bandes, éventuellement dans lequel les parties de matériau rigide sont disposées de manière à ne pas d'empêcher le changement de configuration dans des directions autres que la direction de l'épaisseur.

7. Dispositif médical (100) selon la revendication 5, dans lequel le dispositif médical est agencé pour limiter l'expansion dans l'épaisseur du dispositif lors de changements de configuration, grâce au composant fluidique comprenant un canal fluidique (21) s'étendant à l'intérieur du composant fluidique et le composant fluidique comprenant en outre au moins une attache (21b) qui relie les côtés opposés (21c, 21d) du canal fluidique de manière à empêcher ou limiter l'expansion du canal fluidique dans la direction de l'épaisseur du dispositif lors de changements de configuration.

8. Dispositif médical (100) selon l'une quelconque des revendications 6 à 7, dans lequel le composant fluidique comprend une pluralité de chambres gonflables indépendamment, les chambres étant dimensionnées de manière à empêcher ou limiter l'expansion du canal fluidique dans la direction de l'épaisseur du dispositif lors de changements de configuration, éventuellement dans lequel le composant fluidique comprend en outre une soupape de pression disposée fluidiquement entre une première desdites chambres gonflables indépendamment et une seconde desdites chambres gonflables indépendamment, ladite soupape de pression étant configurée de telle sorte que le fluide ne passe pas de la première chambre à la seconde chambre jusqu'à ce qu'une pression de fluide prédéterminée soit atteinte dans la première chambre.

9. Dispositif médical (100) selon la revendication 2, ou toute revendication dépendant de la revendication 2, comprenant en outre :
un connecteur fluidique (102) en communication fluidique avec le composant fluidique (20) et un connecteur électrique (101) en contact électrique avec le réseau d'électrodes (10), lesdits connecteurs étant disposés dans la section proximale (120, 130) du dispositif pour la connexion du composant fluidique et du réseau d'électrodes à des dispositifs externes, éventuellement dans lequel
la section distale (110) du dispositif est plus flexible que la section proximale (120, 130), et/ou
la section distale (110) comprend au moins 90 % du volume du dispositif.

10. Dispositif médical (100) selon la revendication 9, comprenant en outre un connecteur conducteur reliant le réseau d'électrodes (10) au connecteur électrique (101) et une première gaine (200) qui entoure le connecteur conducteur, comprenant éventuellement en outre un canal de fluide reliant le composant fluidique (20) au connecteur fluidique (102), la première gaine (200) entourant également le canal fluidique.

11. Dispositif médical (100) selon la revendication 10, comprenant en outre une seconde gaine amovible (300) entourant le réseau d'électrodes flexibles (10), le composant fluidique (20) et la première gaine (200), éventuellement dans lequel :
le réseau d'électrodes flexibles (10) et le composant fluidique (20) sont disposés dans une configuration compressée à l'intérieur de la seconde gaine (300), et le dispositif est agencé de telle sorte que l'actionnement du composant fluidique après le retrait de la seconde gaine amène le composant fluidique et le réseau d'électrodes flexibles à passer à une configuration étendue ayant une surface projetée plus grande que la configuration compressée ; et/ou
le diamètre interne de la seconde gaine (300) est de 1 cm ou moins, éventuellement de 5 mm ou moins, et éventuellement de 2 mm ou moins.

12. Dispositif médical (100) selon l'une quelconque des revendications précédentes, dans lequel le composant fluidique (20) et le réseau d'électrodes flexibles (10) sont séparés ou séparables.

13. Dispositif médical (100) selon l'une quelconque des revendications précédentes, dans lequel le dispositif médical comprend un ou plusieurs composants qui peuvent être visualisés par rayons X.

14. Dispositif médical (100) selon l'une quelconque des revendications précédentes, dans lequel le réseau d'électrodes flexibles (10) comprend des électrodes (11) prévues sur un substrat flexible (30).

15. Dispositif médical (100) selon l'une quelconque des revendications précédentes, dans lequel le composant fluidique comprend un canal fluidique (21) qui présente :
une largeur maximale non gonflée de 5 mm ou moins, éventuellement de 3 mm ou moins, en outre éventuellement 1 mm ou moins, en outre éventuellement 500 µm ou moins, en outre éventuellement 100 µm ou moins, en outre éventuellement 50 µm ou moins et encore éventuellement 5 µm ou moins ; et/ou
une épaisseur gonflée maximale de pas plus de 5 mm, éventuellement de pas plus de 2 mm, en outre éventuellement de pas plus de 1 mm, et encore éventuellement de pas plus de 500 µm.
